(19)

Europäisches
Patentamt
European
Patent Office
Office européen
des brevets

(11) **EP 3 859 743 A1**

(12) **EUROPEAN PATENT APPLICATION**

(43) Date of publication:
**04.08.2021 Bulletin 2021/31**

(51) Int Cl.:
***G16H 30/20*** *(2018.01)*  ***G16H 50/20*** *(2018.01)*

(21) Application number: **20154094.5**

(22) Date of filing: **28.01.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
- **SAALBACH, Axel
  5656 AE Eindhoven (NL)**
- **MAVROEIDIS, Dimitrios
  5656 AE Eindhoven (NL)**
- **NICKISCH, Hannes
  5656 AE Eindhoven (NL)**

(74) Representative: **Philips Intellectual Property &
Standards
High Tech Campus 5
5656 AE Eindhoven (NL)**

(54) **UNCERTAINTY-BASED REPRIORITIZATION OF MEDICAL IMAGES BASE UPON CRITICAL FINDINGS**

(57)     A system and method for prioritizing a set of medical images to be evaluated using a machine learning model, including: training the machine learning model using a training data set, wherein the machine learning model receives input medical images and outputs a medical condition shown in the input medical images; running the trained machine learning model on the set of medical images to be evaluated to produce a medical condition output for each of the set of medical images; calculating a likelihood score for each medical condition outputs based upon a determined statistical parameters for the different outputs of the machine learning model; and determining the order of the set of input images to be evaluated based upon the calculated likelihood score and a severity of the medical condition outputs.

FIG. 2

Printed by Jouve, 75001 PARIS (FR)

**Description**

FIELD OF THE INVENTION

Various exemplary embodiments disclosed herein relate generally to a uncertainty-based reprioritization of medical images based upon critical findings.

BACKGROUND OF THE INVENTION

Life-threatening conditions (e.g., pneumothorax) require immediate medical attention in order to prevent significant harm to the patient including death. Therefore, quick communication of findings indicating a server condition is mandated by various medical authorities.

Historically, prioritization of medical image exams has been performed manually or by means of rule-based approaches. However, because of ambiguous labeling schemes, as well as the lack of a stand set of labels to use, the selection of the most important medical images for a radiologist to review has not been effective. Machine learning X-ray systems have been developed, which allow for the automated detection of critical conditions.

SUMMARY OF THE INVENTION

A summary of various exemplary embodiments is presented below. Some simplifications and omissions may be made in the following summary, which is intended to highlight and introduce some aspects of the various exemplary embodiments, but not to limit the scope of the invention. Detailed descriptions of an exemplary embodiment adequate to allow those of ordinary skill in the art to make and use the inventive concepts will follow in later sections.

Various embodiments relate to a method for prioritizing a set of medical images to be evaluated using a machine learning model, including: training the machine learning model using a training data set, wherein the machine learning model receives input medical images and outputs a medical condition shown in the input medical images; running the trained machine learning model on the set of medical images to be evaluated to produce a medical condition output for each of the set of medical images; calculating a likelihood score for each medical condition outputs based upon a determined statistical parameters for the different outputs of the machine learning model; and determining the order of the set of input images to be evaluated based upon the calculated likelihood score and a severity of the medical condition outputs.

Further various embodiments relate to a system for prioritizing a set of medical images to be evaluated using a machine learning model, including: a memory; a processor connected to the memory, the processor configured to: train the machine learning model using a training data set, wherein the machine learning model receives input medical images and outputs a medical condition shown in the input medical images; run the trained machine learning model on the set of medical images to be evaluated to produce a medical condition output for each of the set of medical images; calculate a likelihood score for each medical condition outputs based upon a determined statistical parameters for the different outputs of the machine learning model; and determine the order of the set of input images to be evaluated based upon the calculated likelihood score and a severity of the medical condition outputs.

Various embodiments are described, further including displaying the order of the set of input images on a display for evaluation.

Various embodiments are described, wherein the statistical parameters include a predictive value of the outputs, an uncertainty of the outputs, and the standard deviation of noise.

Various embodiments are described, wherein the likelihood score is calculated as

$$s_y = \frac{\mu_y}{\sqrt{\sigma_y^2 + \sigma_n^2}}$$

wherein $s_y$ is the likelihood score for a specific output $y$, $\mu_y$ is the mean value of the specific output $y$, $\sigma_y$ is the standard deviation of the specific output $y$, and $\sigma_n$ is the standard deviation of noise.

Various embodiments are described, wherein determining the order of the set of input images to be evaluated based upon the calculated likelihood score and a severity of the medical condition outputs includes identifying images in the set of input images that have a high likelihood of having a severe medical condition according to the calculated likelihood score, and sorting, from highest to lowest, the identified images based upon their likelihood score and placing the sorted identified images at the top of the order.

Various embodiments are described, wherein determining the order of the set of input images to be evaluated based upon the calculated likelihood score and a severity of the medical condition outputs further includes sorting, from lowest to highest, images without a high likelihood of having a severe medical condition based upon their calculated likelihood score and placing the sorted images after the sorted identified images in the order.

Various embodiments are described, wherein determining the order of the set of input images to be evaluated based upon the calculated likelihood score and a severity of the medical condition outputs includes identifying images in the set of input images that have a high likelihood of having a severe medical condition according to the calculated likelihood score, calculating an evaluation score based upon the likelihood score and a severity score, wherein the severity score indicates the severity of the medical conditions, and sorting, from highest to lowest, the determined images based upon their evaluation score and placing the determined images at the top of the order.

Various embodiments are described, wherein determining the order of the set of input images to be evaluated based upon the calculated likelihood score and a severity of the medical condition outputs includes identifying images in the set of input images that have a high likelihood of having a severe medical condition according to the calculated likelihood score, determining which of the identified images have a likelihood score above a threshold value, and sorting, from highest to lowest, the determined images based upon their likelihood score and placing the determined images at the top of the order.

Various embodiments are described, wherein determining the order of the set of input images to be evaluated based upon the calculated likelihood score and a severity of the medical condition outputs further includes placing the identified images with a likelihood score below the threshold value after the determined images, and sorting, from lowest to highest, images with a likelihood score below the threshold value and placing the sorted images after the identified images with a likelihood score below the threshold value in the order.

Various embodiments are described, wherein determining statistical parameters for the different output of the machine learning model includes training the machine learning model, and inputting a training data set into a plurality of trained instances of the machine learning model, wherein each of the plurality of trained instances of the machine learning model uses different dropout parameters, and performing a statistical analysis on the outputs from the plurality of trained instances of machine learning models.

BRIEF DESCRIPTION OF THE DRAWINGS

In order to better understand various exemplary embodiments, reference is made to the accompanying drawings, wherein:

> FIG. 1 illustrates an embodiment of system for receiving, storing, and evaluating medical images;
> FIG. 2 illustrates a method that may be used by the medical image controller of FIG. 1 to prioritize unread medical images;
> FIG. 3 illustrates a potential application of the method of FIG. 2 where a certainty threshold is employed; and
> FIG. 4 illustrates an exemplary hardware diagram for implementing the method of FIG. 2 as well as implementing various parts of the system.

To facilitate understanding, identical reference numerals have been used to designate elements having substantially the same or similar structure and/or substantially the same or similar function.

DETAILED DESCRIPTION OF EMBODIMENTS

The description and drawings illustrate the principles of the invention. It will thus be appreciated that those skilled in the art will be able to devise various arrangements that, although not explicitly described or shown herein, embody the principles of the invention and are included within its scope. Furthermore, all examples recited herein are principally intended expressly to be for pedagogical purposes to aid the reader in understanding the principles of the invention and the concepts contributed by the inventor(s) to furthering the art and are to be construed as being without limitation to such specifically recited examples and conditions. Additionally, the term, "or," as used herein, refers to a non-exclusive or (*i.e.,* and/or), unless otherwise indicated (*e.g.*, "or else" or "or in the alternative"). Also, the various embodiments described herein are not necessarily mutually exclusive, as some embodiments can be combined with one or more other embodiments to form new embodiments.

Backlogs of unexamined medical images, such as X-rays, MRI's, ultrasounds, etc. are the result of increase workload for radiologists and shortages of radiologists. Such issues are found in public health care systems. Because of increasing workload, radiologists are pressured to spend less time reviewing each medical exam which cannot be further improved by various existing radiology systems.

This situation may lead to delays in communicating the detection of critical conditions to the referring physician, which may lead to delays in providing critical care that will decrease the likelihood of death or other sever consequences for patients. According, requirements on timely reporting of the detection of critical conditions in medical images has been specified by various medical organizations.

Today various approaches are used to process medical image worklists for radiologists. Some simple process them in the order they are received, with others may use some sort of rules based method of prioritizing the medical images. Because of inconsistent use of the terms to indicate the urgency of examining a specific medical image, such systems often do not properly prioritize the medical images to be evaluated.

Various work has been done to automatically classify medical images using convolutional neural networks (CNN). Such approaches have shown positive results in classifying medical images, and such models may be used to facilitate improving the workflow of radiologists and to help prioritize the review of images in order to more quickly evaluate images that may indicate that a patient has a critical condition.

Nowadays, the performance of many machine learning systems are already highly accurate and highly competitive with human evaluators. However, deep learning techniques such as CNNs do not take the uncertainty of their predictions into account. At the same time, a low specificity can have a substantial negative impact. Especially for conditions with low incidence rates, a low specificity could result in frequent false alarms, reducing not only the clinical acceptance but potentially delaying the time to read for patients with undetected critical conditions. Especially for triage systems, the reliable identification of critical cases is essential. Therefore, embodiments are described herein that re-prioritize predictions with a high predictive uncertainty.

The embodiments described below will focus on chest X-rays to provide an example embodiment. But, these embodiments are applicable to different types of images from various imaging systems (e.g., X-ray, CT, magnetic resonance imaging (MRI), ultrasound, etc.) and workstations.

While deep learning techniques have evolved as the state of the art method for image processing, they do not provide information about the uncertainty of the prediction (as e.g., Bayesian models) without further engineering. The embodiments described herein use recent insights about the relation between CNNs and Gaussian processes in order to reweight the predictions. See Dropout as Bayesian Approximation: Representing Model Uncertainty in Deep Learning, Gal and Ghahramani, https://arxiv.org/abs/1506.02142 which is incorporated herein by reference for all purposes.

The embodiments described herein may be implemented in software, which allows for the detection of critical conditions or other findings, either on the imaging modality or for worklist prioritization. It may employ machine learning technology such as CNNs to predict the probabilities for specific medical conditions.

Using such an approach, a model could indicate a critical condition, but at the same time, the prediction can be associated with a very high uncertainty. In the context of a triage solution, which aims at a low false positive rate, the uncertainty could be used to sort the images for evaluation.

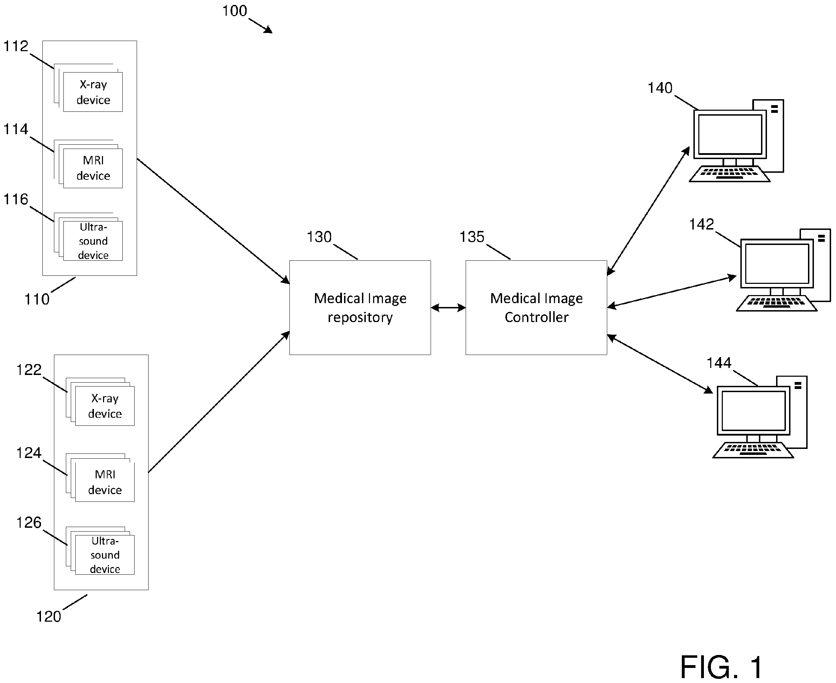

FIG. 1 illustrates an embodiment of system for receiving, storing, and evaluating medical images. The system 100 includes a medical image repository 130 that receives medical images. The medical image repository 130 may be any digital storage capable of storing medical images including, e.g., hard disk drives, solid state storage devices, etc. The medical image repository 130 may receive images from various sources. For example a first medical site 110 and a second medical site 120 may each have various equipment that capture medical images. The first and second medical sites 110, 120 may be remote from the medical image repository 130. The communication between the first and second medical sites 110, 120 and the medical image repository 130 may be done using various networking technologies, such as for example, the internet, local area networks (LAN), wide area networks (WAN), WiFi, various types of cellular communication, etc. The first and second medical sites 110, 120 may have various types of imaging systems, such as for example, X-ray devices 112, 122, MRI devices 114, 124, and ultra-sound devices 116, 126. Other types of medical imaging devices may be present as well. Further, each site may have more than one of any type of medical imaging device.

The system 100 may also include a medical image controller 135. The medical image controller 135 accesses unread medical images from the medical image repository 130 and processes the unread medical images to prioritize them for evaluation by a medical professional. The medical image controller 135 may then send a prioritized list of medical images to various user workstations 140, 142, 144 for medical professional's to evaluate. The user workstations 140, 142, 144 include the needed processing and storage needed for the medical professional to select, view, manage, evaluate, and then store and/or transmit information related to the evaluation of the unread medical images. Various known and existing user workstations are available that may be used for evaluating the prioritized unread medical images.

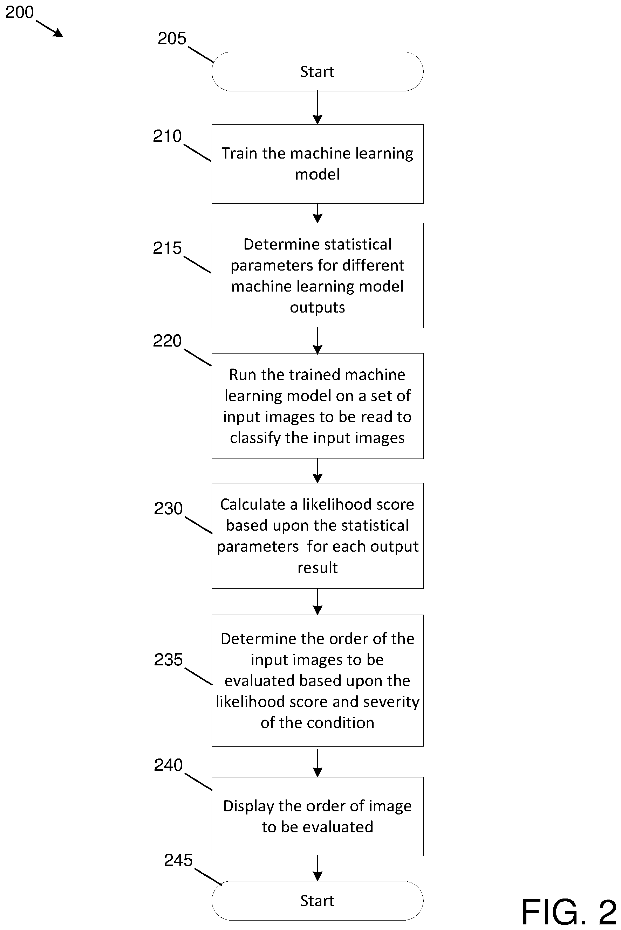

FIG. 2 illustrates a method that may be used by the medical image controller 135 of FIG. 1 to prioritize unread medical images. In the description of the method, chest X-rays will be used as an example of the medical images to be prioritized. Other types of medical images as discussed above may also be analyzed. Further, a machine learning model is used as part of the method, and for this example, a CNN is used as an example machine learning model. But, other types of machine learning models such as support vector machines or random forests may be used as well. The method 200 begins at 205.

Next, the method 200 trains the machine learning model 210. In the current example this means training a CNN that evaluates chest X-rays and specifically looks for pneumothorax, and it may detect a other conditions, e.g., cardiomegaly. The CNN may include a sequence of interconnected layers (e.g., convolutional layer, batch-normalization layer, dense layer) and may be optimized in an iterative training process to predict probabilities for images and relevant medical conditions. Next, the method 200 may determine the statistical parameters for the different machine learning model outputs 215. For example, a model for analyzing chest X-rays may have say 15 different output classifications of the input X-rays. These output values may be continuous values indicating the probability of the presence of a given condition. It is noted that these probabilities do not directly indicate a confidence value for the output. Accordingly, a predicted value of the output, which may be indicted by the mean value of the of each of these different outputs, and an uncertainty value of the output, which may be indicated by the standard deviation for each of these different outputs, may be calculated and then used to determine a likelihood score. One way this may be done is by using a CNN with dropouts. Training a CNN using dropout is a commonly employed technique to prevent overfitting, but it also results in a link to Bayesian inference in deep Gaussian processes that allows for accessing the uncertainty if the outputs(i.e. predictive variance). In particular, using a CNN (trained with dropout), the application of dropout during inference yields individual predictions $y_t$ where t=1..T. These instances may be used to estimate the output mean prediction $\mu_y$, and its standard deviation $\sigma_y$ for each of the model outputs. Other moment-based statistics such as kurtosis or skewness can be estimated, as well. Alternatively to performing dropout, one can simply consider an ensemble of CNNs.

The method 200 then runs the trained machine learning model on a set of input images to be read and classifies the images 220.

The method next calculates a likelihood score for the outputs of the trained machine learning model based upon the statistical parameters determined in step 210. This likelihood score (under a zero mean Gaussian model) may be calculated as follows:

$$s_y = \frac{\mu_y}{\sqrt{\sigma^2_y + \sigma^2_n}}$$

where $s_y$ is the likelihood score for a specific output $y$, $\mu_y$ is the mean value of the specific output $y$, $\sigma_y$ is the standard deviation of the specific output $y$, and $\sigma_n$ is the noise/aleatoric standard deviation rather than noise according to the mean prediction $\mu_y$ alone. A high uncertainty level $\sigma_y$ may render the final value small whereas a small uncertainty $\sigma_y$ may increase the value, which shows the benefit of such a score. Other likelihood scores based on other probabilistic models such as (nonzero mean Gaussian, Laplacian, Cauchy, etc.) may be calculated instead as well.

As described above, the predicted probabilities output from the CNN model do not function as confidence values, and the CNN could predict a high probability while having a high uncertainty. For such cases, automatic prioritization as a critical case would be questionable.

The method 200 then determines the order of the input images to be evaluated based upon the likelihood score and the severity of the condition 235. In one embodiment, the CNN may analyze chest X-rays to detect when pneumothorax is indicated by the X-ray. Pneumothorax may quickly lead to severe consequences for a patient including death, so when pneumothorax is detected it is important that such an X-ray is read a quickly as possible and appropriate treatment ordered. In an embodiment, all X-rays classified has indicating pneumothorax may be determined. Then, all X-rays classified as pneumothorax are sorted, from high to low, based upon their likelihood score. This sorted list is placed at the top of the list of images to be reviewed by a medical professional. This leads to the images having the most likely severe conditions being evaluated first, which reduces the amount of time before treatment may be begun for the associated patient. The remaining images may be placed in any order after the sorted images indicating pneumothorax. In another embodiment, the remaining images may be sorted, from low to high, based upon likelihood score and then placed after the sorted images indicating pneumothorax. In this manner, images that do not indicate a severe conditions but with a low likelihood are placed higher on the list of images to be evaluated. An image that does not indicate a severe condition with a low likelihood score may more likely actually be an image showing a server condition, and hence it is beneficial for a medical professional to evaluate such an image as soon as possible. Further, a normal or non-severe image with a high likelihood score will be among the last images to be evaluated because they are the least likely to indicate a severe condition.

Other embodiments may sort the images in other ways taking into account the severity of the conditions to be evaluated and the likelihood score. For example, if the CNN can detect multiple different serve conditions, any images with these conditions may be sorted based upon their likelihood scores and then placed at the top of the worklist. In another embodiments, there may be for example three different severe conditions, but they are not equal in severity. In such a case, the most severe condition may first be identified, sorted, and placed at the top of the worklist. Next, the images indicate the second most severe condition may be sorted and then place after the first sorted list for the most severe conditions in the worklist. Finally, the third most severe condition may then be sorted and placed on the worklist after the second sorted list. The remaining images may be placed as described above, either as is or further sorted.

In yet another embodiment, there may be for example three different severe conditions, but they are not equal in severity. A severity score may be determined for each condition. Then an evaluation score may be calculated as the product of the severity score and the likelihood score. Then the images indicating the most severe conditions may be sorted based upon this evaluation score. The remaining images may be placed as described above, either as is or further sorted.

Determining the order of the input images may be done in a batch process. That is discrete sets of images are received and then sorted for evaluation by a medical professional. In one embodiment, each batch corresponds to a single medical professional. If more than one medical professional is available to evaluate images, then the sorted list of images is spread among the available medical professionals in a round robin fashion so that the most urgent cases are spread out and can be more quickly evaluated.

In another embodiment, incoming images may be processed in batches or one by one, and added to existing worklists for each medical profession based upon the severity and likelihood. For example, if a medical profession is currently working on a worklist without any severe images, then when one or more severe images are added to the evaluation list they may be added ahead of the existing normal and non-severe images.

Finally, once the images have been placed on a medical professional's worklist, the order of the images to be evaluated may be displayed, and the medical profession may start selecting and evaluating images based upon the displayed worklist 240. The method 200 may then end at 245.

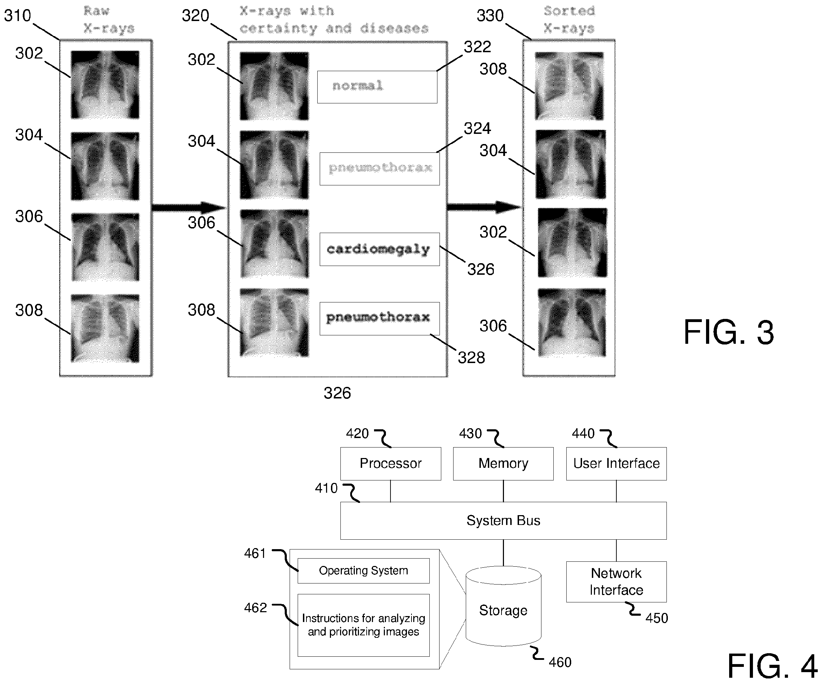

FIG. 3 illustrates a potential application of the method 200 where a likelihood score is employed. As set of input X-rays 310 are shown as ordered in chronological order. In this example, there are four input X-rays 302, 304, 306, and 308. The set of input X-rays 310 are processed by the machine learning model to label the X-rays as shown in 320. The likelihood score is also calculated for each and is indicated by the gray level of the label. For example X-ray 308 is labeled as pneumothorax 328 with a high likelihood (i.e., a black label), while 304 is labeled as pneumothorax 324 but with low likelihood (i.e., with a light gray label). X-ray 306 is labeled as cardiomegaly 326 with a high likelihood. Finally, X-ray 302 is labeled as normal 322 with a medium likelihood. In this example, only the X-ray 308 is labeled as pneumothorax with a high certainty. Therefore, it is ranked highest in the worklist 330. The X-ray 304 contains a treated pneumothorax (with a chest drain - resulting in a high uncertainty), while the remaining X-rays 302, 306 are considered as normal or non-critical. Therefore, only the reliable pneumothorax X-ray 308 is prioritized on the worklist 330 followed by the unreliable pneumothorax X-ray 304 and then the normal or non-critical X-rays.

FIG. 4 illustrates an exemplary hardware diagram 400 for implementing the method of FIG. 2 as well as implementing various parts of the system. As shown, the device 400 includes a processor 420, memory 430, user interface 440, network interface 450, and storage 460 interconnected via one or more system buses 410. It will be understood that FIG. 4 constitutes, in some respects, an abstraction and that the actual organization of the components of the device 400 may be more complex than illustrated.

The processor 420 may be any hardware device capable of executing instructions stored in memory 430 or storage 460 or otherwise processing data. As such, the processor may include a microprocessor, a graphics processing unit (GPU), field programmable gate array (FPGA), application-specific integrated circuit (ASIC), any processor capable of parallel computing, or other similar devices.

The memory 430 may include various memories such as, for example L1, L2, or L3 cache or system memory. As such, the memory 430 may include static random-access memory (SRAM), dynamic RAM (DRAM), flash memory, read only memory (ROM), or other similar memory devices.

The user interface 440 may include one or more devices for enabling communication with a user and may present information such. For example, the user interface 440 may include a display, a touch interface, a mouse, and/or a keyboard for receiving user commands. In some embodiments, the user interface 440 may include a command line interface or graphical user interface that may be presented to a remote terminal via the network interface 450.

The network interface 450 may include one or more devices for enabling communication with other hardware devices. For example, the network interface 450 may include a network interface card (NIC) configured to communicate according to the Ethernet protocol or other communications protocols, including wireless protocols. Additionally, the network interface 450 may implement a TCP/IP stack for communication according to the TCP/IP protocols. Various alternative or additional hardware or configurations for the network interface 450 will be apparent.

The storage 460 may include one or more machine-readable storage media such as read-only memory (ROM), random-access memory (RAM), magnetic disk storage media, optical storage media, flash-memory devices, or similar storage media. In various embodiments, the storage 460 may store instructions for execution by the processor 420 or data upon with the processor 420 may operate. For example, the storage 460 may store a base operating system 461 for controlling various basic operations of the hardware 400. The storage 462 may store instructions for implementing the method of FIG. 2 that analyzes and prioritizes medical images.

It will be apparent that various information described as stored in the storage 460 may be additionally or alternatively stored in the memory 430. In this respect, the memory 430 may also be considered to constitute a "storage device" and the storage 460 may be considered a "memory." Various other arrangements will be apparent. Further, the memory 430 and storage 460 may both be considered to be "non-transitory machine-readable media." As used herein, the term "non-transitory" will be understood to exclude transitory signals but to include all forms of storage, including both volatile and non-volatile memories.

While the host device 400 is shown as including one of each described component, the various components may be duplicated in various embodiments. For example, the processor 420 may include multiple microprocessors that are configured to independently execute the methods described herein or are configured to perform steps or subroutines of the methods described herein such that the multiple processors cooperate to achieve the functionality described herein. Such plurality of processors may be of the same or different types. Further, where the device 400 is implemented in a cloud computing system, the various hardware components may belong to separate physical systems. For example, the processor 420 may include a first processor in a first server and a second processor in a second server.

The medical image prioritizing method and system described herein provides many benefits as described above. The method and system improves the prioritization of medical images to be evaluated by determining a likelihood score along with a classification of conditions found in the X-ray by a machine learning model that is then used to prioritize the input medical images. This system provides a technical improvement in medical image prioritization and can lead to earlier evaluation of images showing severe conditions that will benefit from quicker evaluation and hence lead to earlier treatment of patients with severe conditions.

Any combination of specific software running on a processor to implement the embodiments of the invention, constitute a specific dedicated machine.

As used herein, the term "non-transitory machine-readable storage medium" will be understood to exclude a transitory propagation signal but to include all forms of volatile and non-volatile memory.

Although the various exemplary embodiments have been described in detail with particular reference to certain exemplary aspects thereof, it should be understood that the invention is capable of other embodiments and its details are capable of modifications in various obvious respects. As is readily apparent to those skilled in the art, variations and modifications can be affected while remaining within the spirit and scope of the invention. Accordingly, the foregoing disclosure, description, and figures are for illustrative purposes only and do not in any way limit the invention, which is defined only by the claims.

## Claims

1. A method for prioritizing a set of medical images to be evaluated using a machine learning model, comprising:

   training the machine learning model (210) using a training data set, wherein the machine learning model receives input medical images and outputs a medical condition shown in the input medical images;
   running the trained machine learning model (220) on the set of medical images to be evaluated to produce a medical condition output for each of the set of medical images;
   calculating a likelihood score (230) for each medical condition outputs based upon a determined statistical parameters for the different outputs of the machine learning model; and
   determining the order of the set of input images (235) to be evaluated based upon the calculated likelihood score and a severity of the medical condition outputs.

2. The method of claim 1, further comprising displaying the order of the set of input images on a display for evaluation.

3. The method of claim 1, wherein the statistical parameters include a predictive value of the outputs, an uncertainty of the outputs, and the standard deviation of noise.

4. The method of claim 3, wherein the likelihood score is calculated as

$$s_y = \frac{\mu_y}{\sqrt{\sigma_y^2 + \sigma_n^2}}$$

   wherein $s_y$ is the likelihood score for a specific output $y$, $\mu_y$ is the mean value of the specific output $y$, $\sigma_y$ is the standard deviation of the specific output $y$, and $\sigma_n$ is the standard deviation of noise.

5. The method of claim 1, wherein determining the order of the set of input images to be evaluated based upon the calculated likelihood score and a severity of the medical condition outputs includes identifying images in the set of input images that have a high likelihood of having a severe medical condition according to the calculated likelihood score, and sorting, from highest to lowest, the identified images based upon their likelihood score and placing the sorted identified images at the top of the order.

6. The method of claim 5, wherein determining the order of the set of input images to be evaluated based upon the calculated likelihood score and a severity of the medical condition outputs further includes sorting, from lowest to highest, images without a high likelihood of having a severe medical condition based upon their calculated likelihood score and placing the sorted images after the sorted identified images in the order.

7. The method of claim 1, wherein determining the order of the set of input images to be evaluated based upon the calculated likelihood score and a severity of the medical condition outputs includes identifying images in the set of input images that have a high likelihood of having a severe medical condition according to the calculated likelihood score, calculating an evaluation score based upon the likelihood score and a severity score, wherein the severity score indicates the severity of the medical conditions, and sorting, from highest to lowest, the determined images based upon their evaluation score and placing the determined images at the top of the order.

8. The method of claim 1, wherein determining the order of the set of input images to be evaluated based upon the calculated likelihood score and a severity of the medical condition outputs includes identifying images in the set of input images that have a high likelihood of having a severe medical condition according to the calculated likelihood score, determining which of the identified images have a likelihood score above a threshold value, and sorting, from highest to lowest, the determined images based upon their likelihood score and placing the determined images at the top of the order.

9. The method of claim 8, wherein determining the order of the set of input images to be evaluated based upon the calculated likelihood score and a severity of the medical condition outputs further includes placing the identified images with a likelihood score below the threshold value after the determined images, and sorting, from lowest to highest, images with a likelihood score below the threshold value and placing the sorted images after the identified images with a likelihood score below the threshold value in the order.

10. The method of claim 1, wherein determining statistical parameters for the different output of the machine learning model includes training the machine learning model, and inputting a training data set into a plurality of trained instances of the machine learning model, wherein each of the plurality of trained instances of the machine learning model uses different dropout parameters, and performing a statistical analysis on the outputs from the plurality of trained instances of machine learning models.

11. A system for prioritizing a set of medical images to be evaluated using a machine learning model, comprising:

a memory (430);
a processor (420) connected to the memory, the processor configured to:

train the machine learning model (210) using a training data set, wherein the machine learning model receives input medical images and outputs a medical condition shown in the input medical images;
run the trained machine learning model (220) on the set of medical images to be evaluated to produce a medical condition output for each of the set of medical images;
calculate a likelihood score (230) for each medical condition outputs based upon a determined statistical parameters for the different outputs of the machine learning model; and
determine the order of the set of input images (235) to be evaluated based upon the calculated likelihood score and a severity of the medical condition outputs.

12. The system of claim 11, further comprising a display configured to display the order of the set of input images on a display for evaluation.

13. The system of claim 11, wherein the statistical parameters include a predictive value of the outputs, an uncertainty of the outputs, and the standard deviation of noise.

14. The system of claim 11, wherein determining the order of the set of input images to be evaluated based upon the calculated likelihood score and a severity of the medical condition outputs includes identifying images in the set of input images that have a high likelihood of having a severe medical condition according to the calculated likelihood score, and sorting, from highest to lowest, the identified images based upon their likelihood score and placing the sorted identified images at the top of the order.

15. The system of claim 11, wherein determining the order of the set of input images to be evaluated based upon the calculated likelihood score and a severity of the medical condition outputs includes identifying images in the set of input images that have a high likelihood of having a severe medical condition according to the calculated likelihood score, calculating an evaluation score based upon the likelihood score and a severity score, wherein the severity score indicates the severity of the medical conditions, and sorting, from highest to lowest, the determined images based upon their evaluation score and placing the determined images at the top of the order.

FIG. 1

EP 3 859 743 A1

200

205 — Start

210 — Train the machine learning model

215 — Determine statistical parameters for different machine learning model outputs

220 — Run the trained machine learning model on a set of input images to be read to classify the input images

230 — Calculate a likelihood score based upon the statistical parameters for each output result

235 — Determine the order of the input images to be evaluated based upon the likelihood score and severity of the condition

240 — Display the order of image to be evaluated

245 — Start

FIG. 2

FIG. 3

FIG. 4

Europäisches
Patentamt

European
Patent Office

Office européen
des brevets

**EUROPEAN SEARCH REPORT**

Application Number

EP 20 15 4094

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/156484 A1 (NYE KATELYN ROSE [US] ET AL) 23 May 2019 (2019-05-23) * the whole document * * in particular * * paragraph [0092] - paragraph [0164]; figures 3-9 * | 1-15 | INV. G16H30/20 G16H50/20 |
| X | US 2016/350919 A1 (STEIGAUF WADE J [US] ET AL) 1 December 2016 (2016-12-01) * the whole document * * in particular * * paragraphs 0030, 0035-0043, 0052-0067; figure 7 * | 1-15 | |
| X | US 2019/189267 A1 (STOVAL III WILLIAM MURRAY [US] ET AL) 20 June 2019 (2019-06-20) * the whole document * * in particular * * paragraphs 0013, 0045-0060; figure 4 * | 1-15 | |
| | | | TECHNICAL FIELDS SEARCHED (IPC) |
| X | US 2020/019617 A1 (ESWARAN KRISHNAN [US] ET AL) 16 January 2020 (2020-01-16) * the whole document * * in particular * * paragraphs 0010-0024, 0043-0084; figures 2, 5-7 * | 1-15 | G16H |

-/--

The present search report has been drawn up for all claims

| Place of search | Date of completion of the search | Examiner |
|---|---|---|
| Munich | 1 July 2020 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number

EP 20 15 4094

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZUCKER EVAN J ET AL: "Deep learning to automate Brasfield chest radiographic scoring for cystic fibrosis", JOURNAL OF CYSTIC FIBROSIS, ELSEVIER, NL, vol. 19, no. 1, 2 May 2019 (2019-05-02), pages 131-138, XP086010786, ISSN: 1569-1993, DOI: 10.1016/J.JCF.2019.04.016 [retrieved on 2019-05-02] * the whole document * * in particular * * sections 2, 3; figure 1; table 1 * ----- | 1-15 | |

TECHNICAL FIELDS SEARCHED (IPC)

The present search report has been drawn up for all claims

| Place of search | Date of completion of the search | Examiner |
|---|---|---|
| Munich | 1 July 2020 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**

EP 20 15 4094

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2020

| Patent document cited in search report | | Publication date | Patent family member(s) | | Publication date |
|---|---|---|---|---|---|
| US 2019156484 | A1 | 23-05-2019 | NONE | | |
| US 2016350919 | A1 | 01-12-2016 | US | 2016350919 A1 | 01-12-2016 |
| | | | US | 2018204325 A1 | 19-07-2018 |
| | | | US | 2019279363 A1 | 12-09-2019 |
| US 2019189267 | A1 | 20-06-2019 | NONE | | |
| US 2020019617 | A1 | 16-01-2020 | NONE | | |

EPO FORM P0459

For more details about this annex : see Official Journal of the European Patent Office, No. 12/82